Europäisches Patentamt

**European Patent Office**

Office européen des brevets

(19)

(11) **EP 0 870 499 A1**

(12) **EUROPÄISCHE PATENTANMELDUNG**

(43) Veröffentlichungstag:
**14.10.1998 Patentblatt 1998/42**

(51) Int. Cl.$^6$: **A61K 31/135**
// A61K9/26

(21) Anmeldenummer: **98103874.8**

(22) Anmeldetag: **05.03.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **25.03.1997 DE 19712398**

(71) Anmelder: **Grünenthal GmbH**
**D-52078 Aachen (DE)**

(72) Erfinder:
• **Friderichs, Elmar Josef, Dr.**
**52223 Stolberg (DE)**
• **Winter, Werner, Prof. Dr.**
**52078 Aachen (DE)**

(54) **Orale Anwendung von (+)-0-Demethyltramadol als Schmerzmittel**

(57) Es wird die Verwendung von (+)-O-Demethyltramadol zur oralen Applikation bei der Behandlung von starken akuten und/oder chronischen Schmerzen beschrieben.

EP 0 870 499 A1

**Beschreibung**

Die Erfindung betrifft die orale Anwendung von (+)-O-Demethyltramadol zur Behandlung von starken, akuten und/oder schweren chronischen Schmerzen.

Opioide haben in der Schmerztherapie eine unangreifbare Vormachtsstellung bei der Behandlung von schweren Schmerzen (Zenz, M., Jurna I. (1993) Lehrbuch der Schmerztherapie, Wissenschaftliche Verlagsgesellschaft Stuttgart). Dies gilt für die Behandlung von akuten wie auch chronischen Schmerzzuständen, wobei die opioidtypischen Nebenwirkungen wie Atemdepression, Obstipation und insbesondere die bei chronischer Anwendung auftretende Toleranz- und Abhängigkeitsentwicklung in Kauf genommen werden. Die therapeutische Praxis zeigte, dass oral anwendbare Substanzen für die chronische Schmerzbehandlung besonders gut geeignet sind, da sie leicht dosierbar und im Gegensatz zu Injektionspräparaten von Patient und/oder dein Pflegepersonal ohne besonderen Aufwand und ohne Hilfsmittel appliziert werden können (Cherny, N. J. et al., Medikamentöse Therapie von Tumorschmerzen II, Anwendung von Opioiden, Der Schmerz 9, 3 - 19 (1995), Evans, P. J. Opiates in the Management of Chronic Pain in Clinics and Anastheologic Vol. 1, No. 1, 71 - 94). Voraussetzung ist, dass die Substanzen eine gute orale Verfügbarkeit und eine ausreichend lange Wirkdauer besitzen. Durch eine lange Wirkdauer, die eventuell auch unter Verwendung von galenischen Hilfsmitteln, wie z. B. Depotzubereitungen, erreichbar ist, kann die Applikationshäufigkeit pro Tag reduziert werden, ohne dass es zu einem Einbruch des für eine optimale Therapie erforderlichen gleichmäßigen Wirkspiegels kommt (Strubelt, O., Pharmakologie, Toxikologie und Verschreibung von Opioiden, Internist 35, 185 - 191 (1994).

Für die Behandlung von schweren chronischen Schmerzzuständen hat sich in den letzten Jahren oral retardiertes Morphin immer stärker durchgesetzt (Zenz et al. (1989), Retardiertes Morphin zur Langzeittherapie schwerer Tumorschmerzen, Dtsch. med. Wschr. 114, 43 - 47; Zenz et al. (1990), Orale Opiattherapie bei Patienten mit „nicht-malignen" Schmerzen, Der Schmerz 4, 14; Ventafridda et al. (1990), Side-effects and Limits of Long-term Oral Morphin Therapy, in: Treatment of Chronic Pain pp 256 - 269, M. Mumenthaler, P.A. van Zwieten, J. M. Falcot Eds.: Harwood Academic Publishers, Chur (1990)), so dass orale Morphinpräparate heute die Mittel der Wahl darstellen, obwohl Morphin eine Reihe von ungünstigen Eigenschaften besitzt. Ein wesentlicher Nachteil von Morphin besteht in der geringen oralen Verfügbarkeit, die auf einer starken First-pass-Inaktivierung in der Darmwand und bei der Leberpassage beruht. Alters- oder krankheitsbedingte Änderungen in der Metabolisierungskapazität können daher die orale Verfügbarkeit von Morphin verändern und spezielle Dosisanpassungen erforderlich machen, um Fehldosierungen zu vermeiden. Eine weitere negative Eigenschaft von Morphin ist die starke opstipierende Nebenwirkung, die bei der oralen Morphintherapie zu Verstopfung führt und obligat den Einsatz von Laxantien erforderlich macht (Cherny et al. 1995 a.a.O.). Eine weitere gravierende Nebenwirkung ist das Auftreten von Toleranz und körperlicher sowie psychischer Abhängigkeit, die eine Unterstellung von Morphin unter die Betäubungsmittelgesetzgebung bedingt.

Die geringe orale Verfügbarkeit von Morphin erfordert eine besondere galenische Aufbereitung, z. B. in retardierenden Formulierungen, um Morphin oral applizierbar zu machen. Diese geringe orale Applizierbarkeit ist insbesondere auf die phenolische Hydroxylgruppe des Morphins zurückzuführen. Auch für viele andere morphinanaloge Verbindungen, wie Nalbuphin, Naloxon, Naltrexon, die ebenfalls eine phenolische Hydroxylgruppe aufweisen, wurde eine geringe orale Anwendbarkeit nachgewiesen.

Die der Erfindung zugrunde liegende Aufgabe bestand deshalb in der Bereitstellung einer Verbindung, die in einem Arzneimittel bei starken akuten und/oder chronischen Schmerzen oral applizierbar ist und die eine mit Morphin vergleichbare Wirkstärke aufweist, ohne dessen Nebenwirkungen zu besitzen.

Überraschenderweise wurde gefunden, dass das (+)-Enantiomer des O-Demethyltramadol und seine physiologisch verträglichen Salze eine hervorragende Wirkstärke besitzen und demzufolge eine hohe analgetische Wirkung aufweisen und trotz einer phenolischen Hydroxylgruppe wesentlich besser oral verfügbar als bekannte opioidanaloge Verbindungen sind. Damit wird eine geringere Applikationsdosis verbunden mit einer größeren Dosierungssicherheit ermöglicht.

Darüber hinaus haben tierexperimentelle Befunde gezeigt, dass das (+)-Demethyltramadol schwächere obstipierende Wirkungen als Morphin besitzt. Außerdem ist die Gefahr, daß bei einer Dauerbehandlung eine psychische Abhängigkeit ausgebildet wird, geringer als bei Morphin.

Die gute orale Verfügbarkeit und analgetische Wirksamkeit des als Wirkstoff in einem Arzneimittel erfindungsgemäß verwendeten (+)-O-Demethyltramadols wurde in Analgesietests an Maus und Ratte nachgewiesen.

Die pharmakologischen Untersuchungen wurden wie folgt geführt:

Analgesieprüfung im Writhing-Test an der Maus

Die Untersuchung auf analgetische Wirksamkeit wurde im Phenylchinon-induzierten Writhing an der Maus (modifiziert nach I. C. Hendershot, J. Forsaith, J. Pharmacol. Exp. Ther. 125, 237 - 240 (1959)) durchgeführt. Dazu wurden männliche NMRI-Mäuse mit einem Gewicht von 25 bis 30 g verwendet. Gruppen von 10 Tieren pro Substanzdosis erhielten 10 Minuten nach intravenöser Gabe und 30 Minuten nach oraler Gabe der erfindungsgemäß zu verwendenden Verbindungen 0,3 ml/Maus einer 0,02 %-igen wäßrigen Lösung des Phenylchinon (Phenylbenzochinon, Fa. Sigma, Deisenhofen; Herstellung der Lösung unter Zusatz von 5 % Ethanol und Aufbewahrung im Wasserbad bei 45° C) intraperitoneal appliziert. Die Tiere wurden einzeln in Beobachtungskäfige gesetzt und mittels Drucktastenzähler die Anzahl der Schmerz-induzierten Streckbewegungen (sogenannte Writhing-Reaktionen = Durchdrücken des Körpers nach Abstrecken der Hinterextremitäten) 5 bis 20 Minuten nach der Phenylchinongabe ausgezählt. Aus der dosisabhängigen Abnahme der Writhing-Reaktionen im Vergleich zu parallel untersuchten, nur mit Phenylchinon behandelten Kontrollgruppen wurden mittels Regressionsanalyse (Auswerteprogramm Martens, EDV-Serivce, Eckental) der $ED_{50}$-Wert für die Hemmung der Writhing-Reaktlon berechnet.

Analgesieprüfung im Tail-Flick-Test an Ratte und Maus

Die analgetische Wirksamkeit der erfindungsgemäß zu verwendenden Verbindung wurde im Wärmestrahl (Tail-Flick-Test) an Maus und Ratte nach der Methode von D'Amour und Smith (J. Pharm. Ex. Ther. 72, 74 - 79 (1941)) untersucht. Dazu wurden männliche NMRI-Mäuse mit einem Gewicht von 20 bis 25 g oder weibliche Sprague Dawly-Ratten mit einem Gewicht von 120 bis 160 g verwendet. Die Tiere wurden einzeln in Testkäfige gesetzt und die Schwanzbasis einem fokussierten Wärmestrahl einer elektrischen Lampe (Rhema-Analgesiemeter) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 3 bis 6 Sekunden betrug. Vor Gabe der erfindungsgemäß zu verwendenden Verbindung wurden die Tiere innerhalb von 5 Minuten zweimal getestet und der Mittelwert dieser Messungen als Vortestmittelwert berechnet. Die Schmerzmessung wurde 20, 40 und 60 Minuten nach intravenöser und 30, 60, 90, 120 Minuten nach oraler Gabe durchgeführt. Bei Anstieg der Schmerzlatenz wurde die maximale Expositionszeit auf 12 Sekunden beschränkt und die Zunahme der Latenzzeit von >150 % des Vortestmittelwerts als analgetische Wirkung gewertet. Zur Bestimmung der Dosisabhängigkeit wurden die Verbindungen in 3 bis 5 logarithmisch ansteigenden Dosen, die jeweils die Schwellen- und die maximale Wirkdosis einschlossen, appliziert und aus der Anzahl der Tiere, die analgetische Wirkung zeigten, nach der Methode von Litchfeld und Wilcoxon (J. Pharm. Ex. Ther. 96, 99 bis 113 (1949)) die $ED_{50}$-Werte bestimmt. Die $ED_{50}$-Berechnung erfolgte im Wirkmaximum jeweils 20 Minuten nach intravenöser und jeweils 30 Minuten nach oraler Substanzgabe.

Die Bestimmung der oralen Wirkverfügbarkeit erfolgte aus dem Verhältnis der $ED_{50}$-Werte bei i.v.- und p.o.-Gabe.

$$\text{Wirkverfügbarkeit} = \frac{ED_{50}\ \text{i.v.}}{ED_{50}\ \text{p.o.}}$$

Tabelle 1

| Analgetische Wirksamkeit und orale Wirkverfügbarkeit von Morphin und (+)-O-Demethyltramadol | | |
|---|---|---|
| Versuchsmodell | $ED_{50}$-Werte (mg/kg) | |
| | (+)-O-Demethyltramadol | Morphin |
| Tail-flick, Maus i.v. | 1,41 | 1,05 |
| Tail-flick, Maus p.o. | 3,75 | 18,90 |
| orale Wirkverfügbarkeit | 0,38 | 0,06 |
| Writhing, Maus i.v. | 0,49 | 0,33 |
| Writhing, Maus p.o. | 1,87 | 4,70 |
| orale Wirkverfügbarkeit | 0,26 | 0,07 |
| Tail-flick, Ratte i.v. | 1,01 | 0,93 |
| Tail-flick, Ratte p.o. | 4,90 | 75,00 |
| orale Wirkverfügbarkeit | 0,21 | 0,01 |

Das erfindungsgemäß verwendendete (+)-Demethyltramadol zeigte im Writhing-Test an der Maus und im Tail-Flick-Test an der Ratte eine ausgeprägte analgetische Wirkung. Wahrend bei Morphin der Faktor für die orale Wirkverfügbarkeit an der Maus 0,06 im Tail-Flick und 0,07 im Writhing und an der Ratte 0,01 im Tail-Flick beträgt, ist bei (+)-O-Demethyltramadol der Wert für die Wirkverfügbarkeit an der Maus im Tail-Flick 0,38, im Writhing 0,26 und an der Ratte 0,21. Damit ist die erfindungsgemäß zu verwendende Verbindung an der Maus etwa 5mal besser und an der Ratte etwa 20mal besser oral verfügbar als Morphin und damit wesentlich besser als Morphin für eine orale Schmerztherapie geeignet.

Bei der erfindungsgemäßen Verwendung des (+)-O-Demethyltramadol als analgetischer Wirkstoff in einem Arzneimittel sind darin neben dem (+)-Enantiomer und/oder mindestens eines physiologisch verträglichen Salzes von (+)-O-Demethyltramadol Trägermaterialien, Füllstoffe, Lösungsmittel, Verdünnungsmittel, Farbstoffe und/oder Bindemittel enthalten. Bei den oral anwendbaren Zubereitungsformen können das (+)-O-Demethyltramadol sowohl als freie Base als auch als Salz von anorganischen und organischen Säuren, beispielsweise aromatische Carbonsäuren wie Benzoesäure, Phenylessigsäure, eingesetzt werden. Besonders bevorzugt sind die Salze von lipophilen organischen Säuren, beispielsweise von $C_8$-$C_{22}$ Fettsäuren wie Stearinsäure, Palmitinsäure. Die mit lipophilen organischen Säuren gebildeten Salze des (+)-O-Demethyltramadols sind insbesondere für Retardformen von Arzneimitteln geeignet, die die erfindungsgemäß zu verwendende Verbindung verzögert freisetzen.

(+)-O-Demethyltramadol wird in Retardformen vorzugsweise als Matrixtablette verwendet. Als Matrixbildner eignen sich insbesondere Celluloseether und/oder Celluloseester, die in einer 2 Gew.-%-igen wäßrigen Lösung bei 20° C eine Viskosität zwischen 10.000 und 150.000 mPas besitzen. Besonders geeignete pharmazeutisch akzeptable Matrixbildner sind ausgewählt aus der Gruppe der Methylhydroxypropylcellulosen, Hydroxyethylcellulosen, Hydropropylcellulosen, Methylcellulosen, Ethylcellulosen und Carboxymethylcellulosen und insbesondere ausgewählt aus der Gruppe der Methylhydroxypropylcellulosen, Hydroxyethylcellulosen und Hydroxypropylcellulosen.

In dem zu verwendenden Arzneimittel liegt der verzögert freizusetzende Wirkstoffgehalt vorzugsweise zwischen 10 und 85 Gew.-% und der Gehalt an pharmazeutisch akzeptablem Matrixbildner zwischen 10 und 40 Gew.-%. Besonders bevorzugt werden Arzneimittel mit einem verzögert freizusetzenden Wirkstoffgehalt zwischen 25 und 70 Gew.-% und einem Gehalt an pharmazeutisch akzeptablem Matrixbildner zwischen 10 und 40 Gew.-%.

In den erfindungsgemäß zu verwendenden Arzneimitteln können als weitere Bestandteile pharmazeutisch gebräuchliche Hilfsstoffe wie Füllstoffe, beispielsweise Lactose, mikrokristalline Cellulose oder Calciumhydrogenphosphat, sowie Gleit-, Schmier- und Fließregulierungsmittel, beispielsweise hochdisperses Siliciumdioxid, Talkum, Magnesiumstearat und/oder Stearinsäure enthalten sein, deren Gesamtgehalt in der Tablette zwischen 0 und 80 Gew.-%, vorzugsweise zwischen 5 und 25 Gew.-% liegt.

Die an Patienten zu verabreichende Wirkstoffmenge variiert in Abhängigkeit vom Gewicht des Patienten, von der Applikationsart, der Indikation und dem Schweregrad der Erkrankung. Üblicherweise werden 10 bis 400 mg/kg, vorzugsweise 10 bis 200 mg/kg (+)-O-Demethyltramadol appliziert.

Darüber hinaus kann in festen oralen Darreichungsformen das erfindungsgemäß zu verwendende (+)-O-Demethyltramadol als freie Base und/oder in Form eines physiologisch verträglichen Salzes in Pulvern, Granulaten, Pellets, Tabletten, Dragees und Kapseln verwendet werden. Die Wahl der Zusammensetzung und die Herstellungsverfahren für die genannten Arzneiformen können nach den bekannten Vorschriften der pharmazeutischen Technologie erfolgen.

In Tablettenform können sowohl einfache Tabletten als auch überzogene Tabletten, beispielsweise Filmtabletten oder Dragees verwendet werden. Für die überzogenen Tabletten können ein oder mehrere Überzugsschichten verwendet werden.

**Patentansprüche**

1. Verwendung von (+)-O-Demethyltramadol als freie Base und/oder in Form eines physiologisch verträglichen Salzes zur oralen Applikation bei der Behandlung von starken akuten und/oder chronischen Schmerzen.

2. Verwendung nach Anspruch 1 dadurch gekennzeichnet, daß die Salze des (+)-O-Demethyltramadol mit organischen Säuren insbesondere $C_8$-$C_{22}$ Fettsäuren und aromatischen Carbonsäuren gebildet werden.

3. Verwendung von (+)-O-Demethyltramadol nach Anspruch 1 oder 2 in einem Arzneimittel in Tablettenform mit verzögerter Wirkstofffreisetzung.

4. Verwendung nach Anspruch 3, dadurch gekennzeichnet, daß die Tablettenform eine Matrixtablette ist.

5. Verwendung nach Anspruch 3, dadurch gekennzeichnet, daß als pharmazeutisch akzeptabler Matrixbildner mindestens ein Celluloseether und/oder Celluloseester mit einer Viskosität zwischen 3.000 und 150.000 mPas, vorzugsweise 10.000 und 150.000 mPas in einer 2 Gew.-%igen wäßrigen Lösung bei 20° C enthalten ist.

6. Verwendung nach Anspruch 3 bis 5, dadurch gekennzeichnet, daß als pharmazeutisch akzeptabler Matrixbildner mindestens eine Substanz ausgewählt aus der Gruppe Methylhydroxypropylcellulosen, Hydroxyethylcellulosen, Hydroxypropylcellulosen, Methylcellulosen, Ethylcellulosen und Carboxymethylcellulosen verwendet wird.

7. Verwendung nach einem der Ansprüche 3 bis 6, dadurch gekennzeichnet, daß der verzögert freizusetzende Wirkstoffgehalt zwischen 10 und 85 Gew.-%, vorzugsweise 25 und 70 Gew.-% und der Gehalt an pharmazeutisch akzeptablen Matrixbildnern zwischen 10 und 40 Gew.-% liegt.

## EUROPÄISCHER TEILRECHERCHENBERICHT

Europäisches Patentamt

der nach Regel 45 des Europäischen Patent-übereinkommens für das weitere Verfahren als europäischer Recherchenbericht gilt

Nummer der Anmeldung

EP 98 10 3874

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | EP 0 753 506 A (GRÜNENTHAL GMBH) 15.Januar 1997<br>* Seite 2 - Seite 3 *<br>* Seite 7, Zeile 55 - Zeile 58 *<br>* Seite 8, Zeile 3 - Zeile 16 * | 1-3 | A61K31/135<br>//A61K9/26 |
| Y | | 4-7 | |
| Y | DE 43 29 794 A (GRÜNENTHAL GMBH) 9.März 1995<br>* das ganze Dokument * | 4-7 | |
| Y | DE 43 15 525 A (EURO-CELTIQUE S.A.) 17.November 1994<br>* das ganze Dokument * | 4-7 | |
| X,P | EP 0 786 450 A (GRÜNENTHAL GMBH) 30.Juli 1997<br>* Seite 2 Zeilen 14-16, 25-30, 43-45 * | 1,3 | |
| Y | * Seite 3, Zeile 4 - Zeile 18 *<br>* Seite 5; Beispiel 2 *<br>* Seite 6 *<br>* Ansprüche 7,8 * | 2 | |

-/--

RECHERCHIERTE SACHGEBIETE (Int.Cl.6)

A61K

### UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Obwohl die Ansprüche 1-7 sich auf ein Verfahren zur Behandlung des menschlichen/tierischen Körpers beziehen (Artikel 52(4) EPÜ), wurde die Recherche durchgeführt und gründete sich auf die angeführten Wirkungen der Verbindung/Zusammensetzung.

| Recherchenort | Abschlußdatum der Recherche | Prüfer |
|---|---|---|
| DEN HAAG | 6.Juli 1998 | Gac, G |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

**Europäisches**
**Patentamt**

**EUROPÄISCHER**
**TEILRECHERCHENBERICHT**

Nummer der Anmeldung

EP 98 10 3874

| | EINSCHLÄGIGE DOKUMENTE | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | EP 0 546 676 A (MCNEILAB INC.) 16.Juni 1993<br>* Seite 4, Zeile 28 - Zeile 46 *<br>* Seite 5; Beispiel 3 *<br>* Seite 3 Zeilen 10-15, 27-34, 42-49 *<br>--- | 1,3 | |
| Y | FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN STUDIES ON CHEMICAL STRUCTURE AND ANALGETIC ACTIVITY OF PHENYL SUBSTITUTED AMINOMETHYLCYCLOHEXANOLES"<br>ARZNEIMITTEL FORSCHUNG. DRUG RESEARCH, Bd. 28, Nr. 1A, 1.Januar 1978,<br>Seiten 107-113, XP000608150<br>* Seite 110 linke Spalte Punkt 3.1.1 *<br>* Seite 111 rechte Spalte Punkt 3.2.3 *<br>* Seite 112 Tabelle 5 Verbindung L235 *<br>--- | 1 | |
| Y | HENNIES HH ET AL: "Receptor binding, analgesic and antitussive potency of tramadol and other selected opioids."<br>ARZNEIMITTELFORSCHUNG, JUL 1988, 38 (7) P877-80, GERMANY, WEST, XP002070459<br>* das ganze Dokument *<br>--- | 1 | |
| Y | POULSEN ET AL.: "The hypoalgesic effect of tramadol in relation to CYP2D6"<br>CLIN. PHARMACOL. THER. (ST LOUIS),<br>Bd. 60, Nr. 6, 1996,<br>Seiten 636-644, XP002070460<br>* das ganze Dokument *<br>--- | 1 | |

RECHERCHIERTE SACHGEBIETE (Int.Cl.6)

-/--

**Europäisches Patentamt**

**EUROPÄISCHER TEILRECHERCHENBERICHT**

Nummer der Anmeldung

EP 98 10 3874

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| Y | ELSING, B. ET AL: "Achiral and chiral high-performance liquid chromatographic determination of tramadol and its major metabolites in urine after oral administrztion of racemic tramadol" J. CHROMATOGR., BIOMED. APPL., 1993, 612, 223-30, XP002070461 * Seite 223 * * Seite 229 * --- | 1,2 | |
| Y | SEVCIK, JAN ET AL: "Effects of the central analgesic tramadol and its main metabolite, O-desmethyltramadol, on rat locus coeruleus neurons" BR. J. PHARMACOL., 1993, 110, 169-76, XP002070462 * Zusammenfassung * * Seite 175, linke Spalte, Absatz 3 * --- | 1 | RECHERCHIERTE SACHGEBIETE (Int.Cl.6) |
| Y | LAI ET AL.: "Tramadol, M1 metabolite and enentioner affinities for cloned human opioid receptors expressed in transfected HN9.10 neuroblastoma cells" EUR. J. PHARMACOL., Bd. 316, Nr. 2-3, 1996, Seiten 369-372, XP002070463 * das ganze Dokument * ----- | 1 | |

EPO FORM 1503 03.82 (P04C12)